# EUROPÄISCHE PATENTSCHRIFT

(11) **EP 0 472 769 B1**
(45) Veröffentlichungstag und Bekanntmachung des Hinweises auf die Patenterteilung: **25.10.1995**
(21) Anmeldenummer: 90116698.3
(22) Anmeldetag: 30.08.1990
(51) Int. Cl.: A61B 5/117, G06K 9/00, G06K 9/20, G07C 9/00

(54) **Identifikationseinrichtung für Daumen- oder Fingerabdrücke**
Identification means for thumb- or fingerprints
Dispositif d'identification pour empreintes digitales

(43) Veröffentlichungstag der Anmeldung: 04.03.1992
(73) Patentinhaber: MAURER IDENTIFIKATIONSSYSTEME GMBH, D-80634 München (DE)
(72) Erfinder: Maurer, Thomas, D-80634 München (DE)
(74) Vertreter: TER MEER STEINMEISTER & PARTNER GbR

(56) Entgegenhaltungen:
- EP-A- 0 169 496
- EP-A- 0 171 939
- EP-A- 0 361 987
- US-A- 4 120 585

## Beschreibung

Die Erfindung betrifft eine Identifikationseinrichtung für Daumen- oder Fingerabdrücke gemäß dem Oberbegriff des Patentanspruchs 1.

Eine derartige Einrichtung ist bereits aus der EP-A-0 361 987 bekannt. Diese bekannte Identifikationseinrichtung enthält einen feststehenden, transparenten Körper mit einer ersten Fläche, gegen die ein Daumen oder Finger drückbar ist, eine Beleuchtungseinrichtung zum Beleuchten des gegen die erste Fläche drückbaren Daumens oder Fingers durch den transparenten Körper hindurch sowie eine Bildaufnahmeeinrichtung zum Aufnehmen und Verarbeiten eines Bildes des gegen die erste Fläche gedrückten Daumens oder Fingers mit Hilfe von Licht, das an der ersten Fläche total reflektiert worden ist, wobei die Beleuchtungseinrichtung so ausgedehnt ist, daß durch sie ein gegen die erste Fläche gedrückter Daumen oder Finger vollständig beleuchtet wird, die Bildaufnahmeeinrichtung ein zweidimensionales Bildaufnahmearray aufweist und die Beleuchtungseinrichtung sowie die Bildaufnahmeeinrichtung relativ zum transparenten Körper fest positioniert sind, derart, daß sich der Strahlengang zwischen der Beleuchtungseinrichtung und dem transparenten Körper einerseits sowie zwischen dem transparenten Körper und der Bildaufnahmeeinrichtung andererseits nicht ändert.

Ferner ist aus der US-A-4,537,484 eine Identifikationseinrichtung der genannten Art bekannt, die einen feststehenden, transparenten Körper mit einer ersten Fläche, gegen die ein Daumen oder Finger drückbar ist, eine Beleuchtungseinrichtung zum Beleuchten des gegen die erste Fläche drückbaren Daumens oder Fingers durch den transparenten Körper hindurch sowie eine Bildaufnahmeeinrichtung zum Aufnehmen und Verarbeiten eines Bildes des gegen die erste Fläche gedrückten Daumens oder Fingers mit Hilfe von Licht, das an der ersten Fläche total reflektiert worden ist, enthält.

Bei dieser bekannten Identifikationseinrichtung sind die Beleuchtungseinrichtung und die Bildaufnahmeeinrichtung relativ zur ersten Fläche drehbar, damit ein Daumen oder Finger, der auf der ersten Fläche zu liegen kommt, allseitig abgetastet werden kann. Die Abtastung erfolgt darüber hinaus zeilenweise, da zur Abtastung ein lineares Diodenarray verwendet wird.

Diese Einrichtung weist infolge des Drehmechanismus zum Drehen von Beleuchtungseinrichtung und Bildaufnahmeeinrichtung relativ zum transparenten Körper einen komplizierten Aufbau auf und benötigt außerdem infolge der zeilenweisen Abtastung ausgesprochen viel Zeit zur Erstellung eines Abdrucks des Daumens oder Fingers.

Eine weitere Einrichtung der genannten Art ist aus der US-A-4,322,163 bekannt. Auch dort wird ein Daumen oder Finger zeilenweise abgetastet, wozu ein Abfragelichtstrahl mit Hilfe einer geeigneten optischen Ablenkeinrichtung in Längsrichtung des Fingers abgelenkt wird. Auch hier ist der Aufbau ausgesprochen kompliziert, wobei infolge der zeilenweisen Abtastung wiederum relativ viel Zeit zur Erstellung des Abdrucks des Daumens oder Fingers benötigt wird.

Der Erfindung liegt die Aufgabe zugrunde, die Identifikationseinrichtung der eingangs genannten Art so weiterzubilden, daß Daumen- oder Fingerabdrücke über möglichst große Daumen- oder Fingerbereiche einwandfrei erzeugbar sind.

Die Lösung der gestellten Aufgabe ist im kennzeichnenden Teil des Patentanspruchs 1 angegeben. Vorteilhafte Ausgestaltungen der Erfindung sind den Unteransprüchen zu entnehmen.

Nach der Erfindung ist die Bildaufnahmeeinrichtung mit einer zentralen Prozessoreinheit zur Bildverarbeitung, einem Speicher zur Bildspeicherung und einem Monitor zur Bilddarstellung verbunden, wobei ferner die zentrale Prozessoreinhelt beim Abrollen des Daumens oder Fingers auf der ersten Fläche nacheinander automatisch diejenigen senkrecht zur Abrollrichtung liegenden Bildspalten im Speicher speichert, die jeweils im Zentrum eines bei einer jeweiligen Abrollposition des Daumens oder Fingers erhaltenen Teilbildes liegen.

Bei der Erfindung fällt Licht von der Beleuchtungseinrichtung unter einem solchen Winkel auf die erste Fläche, daß es an dieser Fläche totalreflektiert wird. Lediglich an Stellen, an denen Strukturen eines Daumens oder Fingers direkt an der Fläche zu liegen kommen, wird die Totalreflexion unterbrochen, so daß mit Hilfe der Bildaufnahmeeinrichtung ein kontrastreiches Bild des Daumens oder Fingers aufgenommen werden kann. Der Bildkontrast läßt sich noch dadurch erhöhen, daß auf die erste Fläche eine flüssige oder halbflüssige Schicht, z. B. eine Feuchtigkeitscreme, aufgetragen wird, in die dann der Daumen oder Finger hineingedrückt wird.

Aufgrund der großflächigen Beleuchtung des auf der ersten Fläche liegenden Daumens oder Fingers sowie infolge der Verwendung der Bildaufrahmeeinrichtung mit dem zweidimensionalen Bildaufnahmearray für die zweidimensionale Bildaufnahme läßt sich ein Daumen- oder Fingerabdruck sehr schnell aufnehmen und in einfacher Weise weiter elektronisch verarbeiten.

Der transparente Körper kann z. B. eine Platte sein, deren eine Fläche die erste Fläche und deren andere Fläche entspiegelt ist. Das Licht wird in diesem Fall mit Hilfe der Beleuchtungseinrichtung durch die entspiegelte Fläche hindurch in die Platte eingestrahlt und an der anderen Fläche totalreflektiert, gegen die der Daumen oder Finger gedrückt wird. Es tritt dann durch die entspiegelte Fläche wieder nach außen, um auf die Bildaufnahmeeinrichtung aufzutreffen. Die Platte, die z. B. planparallel ausgebildet ist, kann aus Glas oder einem anderen transparenten Material, beispielsweise aus einem Kunststoff, bestehen, der ein ähnliches optisches Verhalten wie Glas aufweist.

Der transparente Körper kann aber auch ein Dreikantprisma sein, dessen Basisfläche die erste Fläche bildet. In diesem Fall tritt Licht durch eine Kathete in das Dreikantprisma hinein und wird an der Basisfläche totalreflektiert. Danach tritt es durch die andere Kathete wieder aus dem Dreikantprimsa aus und trifft auf die Bildaufnahmeeinrichtung auf. Entspiegelte Flächen sind in diesem Fall nicht erforderlich. Auch das Dreikantprisma kann aus Glas oder einem anderen transparenten Material bestehen, das ein ähnliches optisches Verhalten wie Glas aufweist, beispielsweise aus Kunststoff.

Es kann ein Spiegel vorhanden sein das aus dem transparenten Körper austretende Licht zu reflektieren. Hierdurch wird erreicht, daß sich das Bild des Daumens oder Fingerabdrucks um 180° wenden läßt, bevor es von der Bildaufnahmeeinrichtung aufgenommen wird.

Andererseits kann der transparente Körper ein Dreikantprisma sein, dessen eine Dachfläche die erste Fläche bildet. Die erste Fläche ist also eine Kathete des Dreikantprismas, das z. B. gleichschenkelig oder gleichseitig sein kann. Licht wird in diesem Fall über die Hypotenuse eingestrahlt und an der genannten Dachfläche, die die erste Fläche bildet, totalreflektiert. Es erfolgt dann eine erneute Reflexion an der zweiten Dachfläche, bevor das Licht wieder durch die Hypotenuse austritt und auf die Bildaufnahmeeinrichtung fällt. Ein Spiegel zur Bildumkehr ist in diesem Fall nicht erforderlich.

Bei Verwendung des zuletztgenannten Dreikantprismas können X-Y-Bildverzerrungen auftreten, die den Faktor 1,41 erreichen können. Ferner können auch trapezförmige Bildverzerrungen auftreten, die infolge der Schrägstellung der totalreflektierenden Fläche zum optischen Strahlengang verursacht werden. Diese Verzerrungen lassen sich zu einem wesentlichen Teil dadurch kompensieren, daß die Bild- bzw. Sensorebene der Bildaufnahmeeinrichtung (zweidimensionales CCD-Target Device) ebenfalls relativ zur Zentralaches des auf sie auftreffenden Lichts gekippt wird, und zwar so weit, daß wieder ein unverzerrtes Bild erhalten wird. Entsprechendes gilt für die zur Bildaufnahmeeinrichtung gehörende Eingangsoptik. Neben der Kompensation der X-Y-Verzerrungen und der Trapezverzerrungen lassen sich somit auch die durch die Schrägstellung der totalreflektierenden Prismenebene verursachten Tiefenschärfeprobleme lösen.

Die genannten Verzerrungen des Bildes des Daumens oder Fingers, die aufgrund des prismas bzw. seiner Stellung hervorgerufen werden, lassen sich aber auch durch die Prozessoreinheit korrigieren, die mit der Bildaufnahmeeinrichtung verbunden ist. Neben der zentralen Prozessoreinheit zur Bildverarbeitung sind mit der Bildaufnahmeeinrichtung noch ein Speicher zur Bildspeicherung und ein Monitor zur Bilddarstellung verbunden, wie bereits erwähnt. Zur Korrektur der Bildverzerrungen können geeignete Algorithmen herangezogen werden, die die geometrischen Verhältnisse des optischen Strahlengangs und der Abbildungseinrichtungen berücksichtigen.

Ferner ist es möglich, das Bild einer auf der ersten Fläche liegenden Schicht im Bild des Daumen- oder Fingerabdrucks durch SoftwareVerarbeitung zu eliminieren. Die genannte Schicht kann beispielsweise eine Schmutzschicht sein, die durch nicht ausreichende Entfernung der eingangs genannten flüssigen oder halbflüssigen Schicht auf der ersten Fläche erhalten worden ist. Die Eliminerung kann z. B. durch ein Bildsubtraktionsverfahren erfolgen.

Um einen möglichst großen Daumen- bzw. Fingerabdruck erhalten zu können, kann der Daumen bzw. Finger auf der ersten Fläche abgerollt werden. Nach der Erfindung werden daher beim Abrollen des Daumens oder Fingers auf der ersten Fläche nacheinander diejenigen und senkrecht zur Abrollrichtung liegenden Bildspalten gespeichert, die jeweils im Zentrum eines bei einer jeweiligen Abrollposition des Daumens oder Fingers erhaltenen Teilbildes liegen. Die genannten Bildspalten werden jeweils dem digitalislerten Teilbild entnommen, um auf diese Weise das Gesamtbild des Daumens oder Fingers zu erzeugen. Als Zentrum des Teilbildes kann z. B. sein Flächenschwerpunkt oder ein anderer geeigneter Bildpunkt dienen.

Um zu verhindern, daß auf diese Weise unscharfe Bilder erzeugt werden, wenn sich die Abrollbewegung des Daumens oder Fingers umkehrt, wird bei einer derartigen Richtungsumkehr die Speicherung der Bildspalten unterbrochen. Ein Abrollvorgang zur Aufnahme eines Daumen- oder Fingerabdrucks ist also nur in einer Richtung möglich.

Nach einer anderen und sehr vorteilhaften Ausgestaltung der Erfindung ist eine Anzeigeeinrichtung vorhanden, die anzeigt, wann die Abrollung des Daumens oder Fingers nach dem Aufsetzen auf die erste Fläche sowie das Abnehmen des Daumens oder Fingers von dieser Fläche nach beendeter Bilderzeugung erfolgen können. Die Anzeigeeinrichtung kann z. B. eine optische oder akustische Anzeigeeinrichtung sein.

Es hat sich als vorteilhaft erwiesen, den Abtastbereich auf der ersten Fläche in drei Teilbereiche zu unterteilen, und zwar in Abrollrichtung gesehen. In der Mitte befindet sich ein großer Teilbereich, während links und rechts davon kleine Teilbereiche vorhanden sind.

Soll beispielsweise der Abdruck eines Fingers aufgenommen werden, so wird der Finger z. B. im linken Kleinen Teilbereich auf die erste Fläche aufgesetzt. Das Zentrum des dann erhaltenen Teilbildes liegt dann in diesem kleinen Teilbereich, wobei der Finger zunächst ruht. Wird die Ruhestellung durch die Prozessoreinheit festgestellt, was der Fall ist, wenn der Finger über eine vorbestimmte Zeit nicht bewegt wird, so wird nicht nur diejenige Bildspalte gespeichert, die durch das Teilbildzentrum hindurchläuft, sondern zuvor, und zwar beginnend vom äußersten linken Rand, alle anderen Bildspalten links vom Teilbildzentrum. Sobald die zuletzt gespeicherte Bildspalte diejenige ist, die durch das Teilbildzentrum hindurchläuft, erfolgt die Ausgabe eines Signals, so daß jetzt der Finger nach rechts abgerollt werden kann. Dabei werden nacheinander im Zentrum bzw. Flächenschwerpunkt des Fingerbildes liegende Bildspalten gespeichert. Nähert sich der Finger dem rechten Rand, so bleibt er noch eine gewisse Zeit auf der ersten Fläche liegen, bevor er abgehoben werden kann. Die Prozessoreinheit detektiert diese Ruhestellung und speichert nach der letzten im Zentrum bzw. Flächenschwerpunkt des Fingerbildes liegenden Bildspalte auch noch die restlichen und rechts von dieser Bildspalte liegenden anderen Bildspalten bis zum äußerten rechten Rand der ersten Fläche. Danach erfolgt eine zweite Anzeige, so daß jetzt der Finger von dieser Fläche abgehoben werden kann. Entsprechendes gilt in umgekehrter Abrollrichtung. Die Einrichtung arbeitet somit völlig symmetrisch, so daß sie sich sowohl für Rechts- als auch für Linshänder eignet.

Liegt das Zentrum bzw. der Flächenschwerpunkt des Fingerbildes beim Aufsetzen auf die erste Fläche bereits im mittleren Bereich, so werden nur durch das jeweilige Bildzentrum verlaufende Bildspalten gespeichert. In diesem Fall kann ein Alarmsignal ausgegeben werden, da jetzt ein nicht vollständiges Bild des Fingers erhalten wird.

Die Erfindung wird nachfolgend unter Bezugnahme auf die Zeichnung näher beschrieben. Es zeigen:
- Fig. 1: eine Identifikationseinrichtung mit einem plattenförmigen, transparenten Körper,
- Fig. 2: eine Identifikationseinrichtung mit einem Primsa, bei dem die Hypotenuse als Auflagefläche benutzt wird,
- Fig. 3: eine Identifikationseinrichtung mit einem Prisma, bei dem eine Kathete als Auflagefläche benutzt wird,
- Fig. 4: eine Identifikationseinrichtung mit einem Prisma, bei dem eine Kathete als Auflagefläche benutzt wird, und mit einer schräggestellten Bildabtastfläche bzw. Eingangsoptik, und
- Fig. 5: ein digitalisiertes Teilbild eines Daumens oder Fingers.

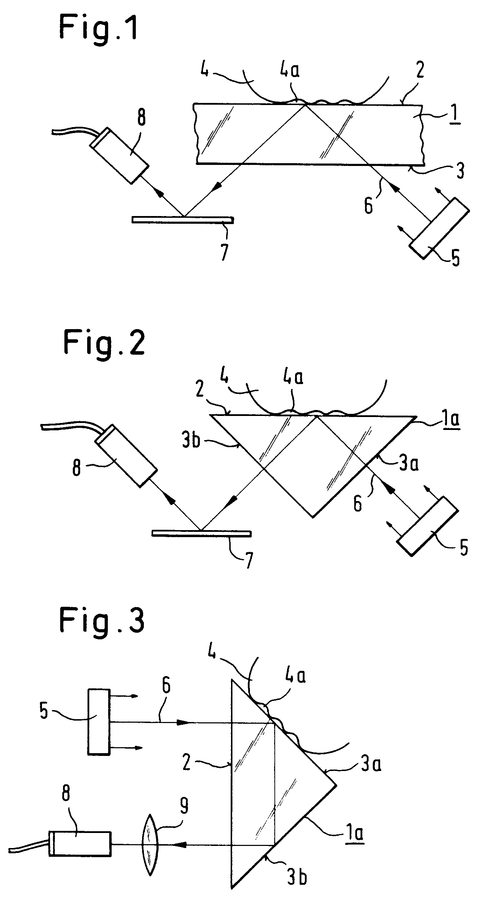

In den Fig. 1 bis 5 sind gleiche Elemente mit den gleichen Bezugszeichen versehen.

Die Fig. 1 zeigt eine erste Ausführungsform einer Identifikationseinrichtung nach der Erfindung. Diese Identifikationseinrichtung enthält eine transparente Glasplatte 1 mit einer ersten Fläche 2 und einer zweiten Fläche 3. Diese Glasplatte 1 ist fest angeordnet, so daß ein Finger 4 auf ihre erste Fläche 2 gedrückt werden kann. Die zweite Fläche 3 ist entspiegelt. Eine ausgedehnte bzw. großflächige Lichtquelle 5 dient zur Aussendung eines Lichtstrahls 6, der durch die entspiegelte zweite Fläche 3 hindurch in die Glasplatte 1 eingestrahlt wird. Der Lichtstrahl 6 wird an der ersten Fläche 2 totalreflektiert und tritt dann durch die zweite Fläche 3 hindurch wieder nach außen aus. Er fällt zunächst auf einen Spiegel 7 und wird an diesem in Richtung einer Bildaufnahmeeeinrichtung 8 reflektiert, die hier eine Videokamera ist. Obwohl nicht im einzelnen dargestellt, ist die Videokamera 8 mit einer zentralen Prozessoreinheit zur Bildverarbeitung, einem Speicher zur Bildspeicherung und einem Monitor zur Bilddarstellung verbunden.

Wie in Fig. 1 zu erkennen ist, werden je nach Oberflächenstruktur des Fingers 4 kleine Hohlräume 4a zwischen Finger 4 und erster Fläche 2 der Glasplatte 1 gebildet, wenn der Finger 4 auf die erste Fläche 2 gedrückt wird. Im Bereich dieser Hohlräume 4a erfolgt eine Totalreflexion des Lichtstrahls 6 an der ersten Oberfläche 2, während dort, wo der Finger 4 direkt in Kontakt mit dieser Oberfläche 2 steht. die Totalreflexion unterbrochen wird. Auf diese Weise wird ein kontraststarkes Bild der Fingeroberfläche (Fingerabdruck) erhalten, das mit Hilfe der Videokamera 8 aufgenommen wird, um anschließend gespeichert und gegebenenfalls verarbeitet zu werden.

Der Spiegel 7 dient zur Umkehrung des Bildes des Fingers 4, damit dieses originalgetreu von der Videokamera 8 aufgenommen werden kann. Das aufgenommene Bild wird gespeichert und kann z. B. zu Identifikationszwecken verwendet werden.

Eine zweite Ausführungsform der erfindungsgemäßen Identifikationseinrichtung ist in der Fig. 2 dargestellt. Gegenüber der Fig. 1 ist dort die transparente Glasplatte durch ein transparentes Dreikantprisma 1a ersetzt worden. Der Lichtstrahl 6 tritt durch eine Kathete 3a in das Dreikantprisma 1a hinein, das z. B. ein gleichseitiges oder gleichschenkliges Prisma aus Glas oder einem transparenten Kunststoff sein kann, der die gleichen optischen Eigenschaften wie Glas aufweist. Er wird dann an der Hypotenuse des Dreikantprismas 1a im Bereich der Ausnehmungen 4a totalreflektiert, wobei die Hypotenuse wiederum die genannte erste Fläche 2 bildet, gegen die der Finger 4 drückbar ist.

Nach Totalreflexion des Lichtstrahls 6 an der Hypotenuse bzw. Basisfläche 2 des Dreikantprismas 1a tritt dieser durch die zweite Kathete 3b nach außen aus und wird wiederum am Spiegel 7 reflektiert, bevor er durch die Eingangsoptik der Videokamera 8 auf deren Bildempfangsbereich trifft. Der Lichtstrahl 6 verläuft vorzugsweise senkrecht zu den Katheten 3a und 3b.

Bei dem in Fig. 3 gezeigten dritten Ausführungsbeispiel der Identifikationseinrichtung nach der Erfindung ist die erste Fläche die Dachfläche bzw. Kathete 3a des Dreikantprismas 1a. Der von der Lichtquelle 5 kommende Lichtstrahl 6 tritt also über die Basisfläche bzw. Hypotenuse 2 in das Dreikantprisma 1a ein und wird zunächst im Bereich der Hohlräume 4a an der Kathete 3a totalreflektiert. Eine weitere Reflexion bzw. Spiegelung erfolgt dann an der zweiten Kathete 3b, bevor das Licht das Prisma 1a durch die Hypotenuse 2 wieder verläßt. Es fällt dann über ein geeignetes optisches Abbildungssystem 9 auf die Videokamera 8 auf.

Infolge der zweifachen Reflexion des Lichtstrahls 6 an den Katheten 3a und 3b ist kein Spiegel 7 zur Bildumkehr mehr erforderlich. Allerdings treten im vorliegenden Fall X-Y-Bildverzerrungen und trapezförmige Bildverzerrungen infolge der Schrägstellung der totalreflektierenden Fläche 3a relativ zum optischen Strahlengang bzw. Lichtstrahl 6 auf. Diese Bildverzerrungen lassen sich jedoch mit Hilfe von Algorithmen auf elektronischem Wege korrigieren, wobei die hierzu erforderliche Software in der zentralen Prozessoreinheit bzw. in dem mit ihr verbundenen Speicher gespeichert ist.

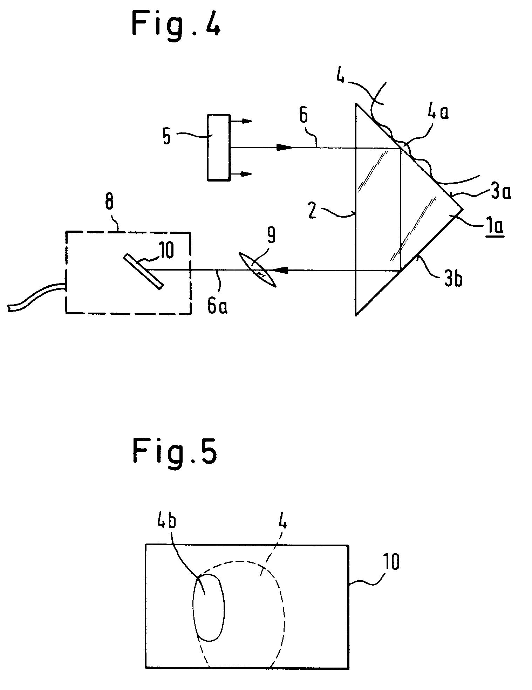

Das Ausführungsbeispiel nach Fig. 4 stimmt mit dem Ausführungsbeispiel nach Fig. 3 im wesentlichen überein, jedoch ist abweichend von Fig. 3 die Sensorebene 10 der Videokamera 8 sowie deren Eingangsoptik 9 relativ zur Zentralachse 6a des auf sie auftreffenden Lichts gekippt. Die Sensorebene 10 ist beispielsweise die CCD-Target-Device-Ebene der Videokamera 8. Durch eine Neigung dieser Sensorebene 10 und der Eingangsoptik 9 relativ zur Zentralachse 6a des auf sie auftreffenden Lichts lassen sich die X-Y-Bildverzerrungen und die trapezförmigen Bildverzerrungen beseitigen. Ferner wird durch die Schrägstellung dieser Elemente auch das durch die Schrägstellung der Totalreflexionsebene bzw. Kathete 3a verursachte Tiefenschärfeproblem gelöst. Eventuelle Restverzerrungen können auch hier auf elektronischem Wege korrigiert werden.

Zur Erzeugung eines Daumen- oder Fingerabdrucks eines möglichst großen Daumen- oder Fingerbereichs kann der Daumen bzw. Finger aber auch auf der ersten Fläche abgerollt werden. Mit Hilfe der Videokamera 8 und der zentralen Prozessoreinheit wird für jede Abrollposition des Daumens oder Fingers ein digitalisiertes Teilbild erzeugt, das in Fig. 5 dargestellt ist. In Fig. 5 ist eine Bildebene mit dem Bezugszeichen 10 versehen, während das digitalisierte Teilbild das Bezugszeichen 4b und der Daumen bzw. Finger das Bezugszeichen 4 tragen. Infolge der Totalreflexion an der ersten Fläche wird nur der Kontaktbereich Zwischen Daumen bzw. Finger und der ersten Fläche abgebildet, so daß das Teilbild 4b entsteht. Der restliche Bereich des Daumens bzw. Fingers 4 bleibt unsichtbar.

Um den gesamten Abdruck zu erhalten, wird von Jedem Teilbild 4b, das im Bildspeicher gespeichert wird, diejenige Bildspalte in einem separaten Speicherbereich gespeichert, die durch das Zentrum des Jeweiligen Teilbildes hindurchläuft und die senkrecht zur Abrollrichtung liegt, die in Fig. 5 in Horizontalrichtung verläuft. Die Bildspalte verläuft in Fig. 5 also in Vertikalrichtung. Das Zentrum eines Teilbildes 4b kann z. B. in dessen Flächenschwerpunkt liegen. Es kann aber auch, in Horizontalrichtung gesehen, zur Bildung des Teilbildzentrums ein geeigneter Bildmittelpunkt ausgewählt werden. Das Zentrum des Teilbildes 4b wird durch geeignete Randfindungs- und Mittelwertalgorithmen ermittelt.

Mit der Abrollbewegung des Daumens oder Fingers verschiebt sich die im Zentrum des Teilbildes liegende Bildspalte, so daß letztlich alle nebeneinanderliegenden Bildspalten gespeichert werden, die zusammengenommen den Daumen- oder Fingerabdruck ergeben.

Um eine Bildverschmierung bei Anderung der Richtung der Abrollbewegung zu vermeiden, wird der Bildaufbau mit Hilfe der genannten senkrechten Bildspalten gestoppt bzw. gelöscht, wenn eine derartige Richtungsänderung eintritt. Der Bilderzeugungsvorgang muß dann wiederholt werden. Hierzu kann ein Benutzer durch die Elektronik aufgefordert werden.

Es sei noch darauf hingewiesen, daß sämtliche Komponenten des erfindungsgemäßen Identifikationssystems in einer einzigen, kompakten Einheit angeordnet sind, ohne daß irgendwelche bewegbaren Teile vorhanden sind. Insbesondere ist auch die Bildaufnahmeeinrichtung fest innerhalb dieser Einheit positioniert, so daß sie einen mechanisch äußerst stabilen Aufbau besitzt.

## Patentansprüche

1. Identifikationseinrichtung für Daumen- oder Fingerabdrücke mit
- einem feststehenden, transparenten Körper (1, 1a) mit einer ersten Fläche (2, 3a), gegen die ein Daumen oder Finger drückbar ist,
- einer Beleuchtungseinrichtung (5) zum Beleuchten des gegen die erste Fläche (2, 3a) drückbaren Daumens oder Fingers durch den transparenten Körper (1, 1a) hindurch und
- einer Bildaufnahmeeinrichtung (8) zum Aufnehmen und Verarbeiten eines Bildes des gegen die erste Fläche gedrückten Daumens oder Fingers mit Hilfe von Licht, das an der ersten Fläche (2, 3a) totalreflektiert worden ist, wobei
- die Beleuchtungseinrichtung (5) so ausgedehnt ist, daß durch sie ein gegen die erste Fläche (2, 3a) gedrückter Daumen oder Finger vollständig beleuchtet wird,
- die Bildaufnahmeeinrichtung (8) ein zweidimensionales Bildaufnahmearray aufweist und
- die Beleuchtungseinrichtung (5) sowie die Bildaufnahmeeinrichtung (8) relativ zum transparenten Körper (1, 1a) fest positioniert sind, derart, daß sich der Strahlengang zwischen der Beleuchtungseinrichtung (5) und dem transparenten Körper (1, 1a) einerseits sowie zwischen dem transparenten Körper (1, 1a) und der Bildaufnameeinrichtung (8) anderseits nicht ändert,
**dadurch gekennzeichnet,** daß die Bildaufnahmeeinrichtung (8) mit einer zentralen Prozessoreinheit zur Bildverarbeitung, einem Speicher zur Bildspeicherung und einem Monitor zur Bilddarstellung verbunden ist, und daß ferner die zentrale Prozessoreinheit beim Abrollen des Daumens oder Fingers auf der ersten Fläche nacheinander automatisch diejenigen senkrecht zur Abrollrichtung liegenden Bildspalten im Speicher speichert, die jeweils im Zentrum eines bei einer jeweiligen Abrollposition des Daumens oder Fingers erhaltenen Teilbildes (4b) liegen.

2. Identifikationseinrichtung nach Anspruch 1, **dadurch gekennzeichnet,** daß der transparente Körper eine Platte (1) ist, deren eine Fläche (2) die erste Fläche und deren andere Fläche (3) entspiegelt ist.

3. Identifikationseinrichtung nach Anspruch 1, **dadurch gekennzeichnet,** daß der transparente Körper ein Dreikantprisma (1a) ist, dessen Basisfläche (2) die erste Fläche bildet.

4. Identifikationseinrichtung nach Anspruch 2 oder 3, **gekennzeichnet durch** einen Spiegel (7) zum Reflektieren des aus dem transparenten Körper (1, 1a) austretenden Lichts.

5. Identifikationseinrichtung nach Anspruch 1, **dadurch gekennzeichnet,** daß der transparente Körper ein Dreikantprisma (1a) ist, dessen eine Dachfläche (3a) die erste Fläche bildet.

6. Identifikationseinrichtung nach einem der Ansprüche 1 bis 5, **dadurch gekennzeichnet,** daß das Bildaufnahmearray (10) der Bildaufnahmeeinrichtung (8) und ihre Eingangsoptik relativ zur Zentralachse (6a) des auf sie auftreffenden Lichts gekippt ist.

7. Identifikationseinrichtung nach Anspruch 1, **dadurch gekennzeichnet,** daß als Zentrum eines Teilbildes (4b) sein Flächenschwerpunkt dient.

8. Identifikationseinrichtung nach Anspruch 1, **dadurch gekennzeichnet,** daß die zentrale Prozessoreinheit bei einer Richtungsumkehr der Abrollbewegung die Speicherung der Bildspalten unterbricht.

9. Identifikationseinrichtung nach einem der Ansprüche 1 bis 8, **dadurch gekennzeichnet,** daß durch die Prozessoreinheit Verzerrungen des Bildes des Daumens oder Fingers, die aufrund der Abbildungsoptik hervorgerufen werden, korrigierbar sind.

10. Identifikationseinrichtung nach einem der Ansprüche 1 bis 9, **dadurch gekennzeichnet,** daß das Bild einer auf der ersten Fläche (2, 3a) liegenden Schicht im Bild des Daumen- oder Fingerabdrucks durch Software-Verarbeitung eliminierbar ist.

11. Identifikationseinrichtung nach einem der Ansprüche 1 bis 10, **gekennzeichnet durch** eine Anzeigeeinrichtung, die anzeigt, wenn die Abrollung des Daumens oder Fingers nach dem Aufsetzen auf die erste Fläche sowie das Abnehmen des Daumens oder Fingers von dieser Fläche nach beendeter Bilderzeugung erfolgen können.

12. Identifikationseinrichtung nach einem der Ansprüche 1 bis 11, **dadurch gekennzeichnet,** daß die Bildaufnahmeeinrichtung (8) eine Videokamera ist.

## Claims

1. Identification device for thumbprints or fingerprints comprising
- a fixed, transparent body (1, 1a) having a first surface (2, 3a) against which a thumb or finger can be pressed,
- an illuminating device (5) for illuminating through the transparent body (1, 1a) the thumb or finger that can be pressed against the first surface (2, 3a), and
- an image recording device (8) for recording and processing an image of the thumb or finger pressed against the first surface with the aid of light that has been totally reflected at the first surface (2, 3a),
- the illuminating device (5) being extended such that a thumb or finger pressed against the first surface (2, 3a) is completely illuminated through it,
- the image recording device (8) having a two-dimensional image recording array, and
- the illuminating device (5) and the image recording device (8) being positioned fixedly relative to the transparent body (1, 1a) in such a way that the beam path does not vary between the illuminating device (5) and the transparent body (1, 1a), on the one hand, or between the transparent body (1, 1a) and the image recording device (8), on the other hand,
**characterized in that** the image recording device (8) is connected to a central processing unit for image processing, a memory for image storage and a monitor for image representation, and in that, moreover, during rolling of the thumb or finger on the first surface the central processing unit automatically sequentially stores in the memory those image columns lying perpendicular to the rolling direction which respectively lie at the centre of a partial image (4b) obtained in the case of a respective rolling position of the thumb or finger.

2. Identification device according to Claim 1, **characterized in that**, the transparent body is a plate (1), of which one surface (2) is the first surface and of which the other surface is antireflection-coated.

3. Identification device according to Claim 1, **characterized in that,** the transparent body is a triangular prism (1a) whose base face (2) forms the first surface.

4. Identification device according to Claim 2 or 3, **characterized in that** a mirror (7) for reflecting the light emerging from the transparent body (1, 1a).

5. Identification device according to Claim 1, **characterized in that,** the transparent body is a triangular prism (1a), one of whose roof faces forms the first surface.

6. Identification device according to one of Claims 1 to 5, **characterized in that,** the image recording array (10) of the image recording device (8) and the entrance optics of the latter is [sic] tilted relative to the central axis (6a) of the light impinging on it.

7. Identification device according to Claim 1, **characterized in that,** the centroid of a partial image (4b) serves as its centre.

8. Identification device according to Claim 1, **characterized in that,** when the direction of the rolling movement is reversed, the central processing unit interrupts the storage of the image columns.

9. Identification device according to one of Claims 1 to 8, **characterized in that,** distortions of the image of the thumb or finger which are caused by the imaging optics can be corrected by means of the processing unit.

10. Identification device according to one of Claims 1 to 9, **characterized in that,** it is possible to eliminate the image of a layer lying on the first surface (2, 3a) in the image of the thumbprint or fingerprint by software processing.

11. Identification device according to one of Claims 1 to 10, **characterized by** a display device which displays when it is possible to perform the rolling of the thumb or finger after its being placed on the first surface, and the lifting of the thumb or finger from this surface after completed image production.

12. Identification device according to one of Claims 1 to 11, **characterized in that,** the image recording device (8) is a video camera.

## Revendications

1. Dispositif d'identification pour empreintes digitales présentant:
- un corps transparent immobile (1, 1a) avec une première surface (2, 3a) contre laquelle un pouce ou un doigt peut être appuyé,
- un dispositif d'illumination (5) pour illuminer le pouce ou le doigt susceptible d'être appuyé contre la première surface (2, 3a), à travers le corps transparent (1, 1a), et
- un dispositif de prise de vue (8) pour enregistrer et pour traiter une image d'un pouce ou d'un doigt appuyé contre la première surface à l'aide d'une lumière qui a subi une réflexion totale sur la première surface (2, 3a) dans lequel
- le dispositif d'illumination (5) présente une extension telle qu'il permet d'illuminer complètement un pouce ou un doigt appuyé contre la première surface (2, 3a),
- le dispositif de prise de vue (8) est muni d'une matrice bidimensionnelle de prise de vue, et
- le dispositif d'illumination (5) ainsi que le dispositif de prise de vue (8) sont positionnés de façon fixe par rapport au corps transparent (1, 1a) d'une telle façon que le trajet des rayons entre le dispositif d'illumination (5) et le corps transparent (1, 1a) d'une part, ainsi qu'entre le corps transparent (1, 1a) et le dispositif de prise de vue (8) d'autre part, ne change pas
caractérisé en ce que le dispositif de prise de vue (8) est relié à une unité de traitement centrale pour le traitement de l'image, à une mémoire pour le stockage de l'image et à un moniteur pour la visualisation de l'image, et en ce que, de plus, l'unité de traitement centrale, lors que le pouce ou le doigt roule sur la première surface, réalise automatiquement le stockage dans la mémoire, l'une après l'autre, de celles des colonnes d'images perpendiculaires à la direction du roulement et qui sont situées dans chaque cas au centre d'une image partielle (4b) obtenue pour une position correspondante de roulement du pouce ou du doigt.

2. Dispositif d'identification selon la revendication 1, caractérisé en ce que le corps transparent est une plaque (1), dont l'une des surfaces (2) constitue la première surface et dont l'autre surface (3) est une surface anti-reflet.

3. Dispositif d'identification selon la revendication 1, caractérisé en ce que le corps transparent est un prisme triangulaire (1a) dont la surface de base (2) constitue la première surface.

4. Dispositif d'identification selon la revendication 2 ou 3, caractérisé par un miroir (7) pour réfléchir la lumière sortant du corps transparent (1, 1a).

5. Dispositif d'identification selon la revendication 1, caractérisé en ce que le corps transparent est un prisme triangulaire (1a), dont un versant (3a) constitue la première surface.

6. Dispositif d'identification selon l'une des revendications 1 à 5, caractérisé en ce que la matrice de prise de vue (10) du dispositif de prise de vue (8) ainsi que son système optique d'entrée sont inclinés relativement à l'axe central (6a) de la lumière qui les frappe.

7. Dispositif d'identification selon la revendication 1, caractérisé en ce que pour le centre d'une image partielle (4b), on prend le barycentre de sa surface.

8. Dispositif d'identification selon la revendication 1, caractérisé en ce que l'unité de traitement centrale interrompt la mise en mémoire des colonnes de l'image dans le cas d'un changement de direction du mouvement de roulement.

9. Dispositif d'identification selon l'une des revendications 1 à 8, caractérisé en ce que des distorsions de l'image du pouce ou du doigt, provoquées par le système optique de formation de l'image, peuvent être corrigées par l'unité de traitement.

10. Dispositif d'identification selon l'une des revendications 1 à 9, caractérisé en ce que l'image, sur la représentation de l'empreinte digitale, d'une couche située sur la première surface (2, 3a) est éliminable par traitement logiciel.

11. Dispositif d'identification selon l'une des revendications 1 à 10, caractérisé par un dispositif indicateur, qui indique le moment où le roulement du pouce ou du doigt préalablement posé sur la première surface peut débuter, ainsi que le moment où le pouce ou le doigt peut être enlevé de cette surface après avoir terminé la génération de l'image.

12. Dispositif d'identification selon l'une des revendications 1 à 11, caractérisé en ce que le dispositif de prise de vue (8) est une caméra vidéo.
